# EUROPEAN PATENT APPLICATION

(11) **EP 4 617 289 A1**
(43) Date of publication of application: **17.09.2025**
(21) Application number: 23888779.8
(22) Date of filing: 09.11.2023
(51) Int. Cl.: C07K 19/00, A61K 38/16, A61K 47/64, A61P 27/02, C07K 14/47, A61K 38/26, C07K 14/605

(54) **PHYSIOLOGICALLY-ACTIVE PEPTIDE DERIVATIVE FOR TREATMENT OF EYE DISEASES, PHARMACEUTICAL COMPOSITION, NASAL/NASAL DROP PREPARATION, AND USE OF PHYSIOLOGICALLY-ACTIVE PEPTIDE DERIVATIVE**

(30) Priority: 10.11.2022 JP 2022180381
(71) Applicant: Glytech, Inc., Kyoto-shi, Kyoto 6008813 (JP)
(72) Inventor: YAMASHITA, Chikamasa, Tokyo 162-8601 (JP)
(74) Representative: Berggren Oy
(86) International application number: PCT/JP2023/040455
(87) International publication number: WO 2024/101434

(57) **Abstract**

A physiologically-active peptide derivative for treatment of an eye disease, the physiologically-active peptide derivative having a physiologically-active peptide sequence and a functional sequence, and the functional sequence including a cell-penetration accelerating sequence and an endosomal-escape accelerating sequence.

## Description

### [Technical Field]

The present invention relates to a physiologically-active peptide derivative for treatment of eye diseases, a pharmaceutical composition, a nasal/nasal drop preparation, and a use of the physiologically-active peptide derivative.

### [Background Art]

Intravitreal injection is a treatment method for eye diseases in which a medicine is injected into the eye ball with a syringe (see, for example, Patent Document 1). For example, injection of an anti-VEGF substance, which has an effect of contracting abnormal blood vessels that grow toward the retina from the choroid that is located underneath the retina (choroidal neovascular vessels), into the eye ball is conducted to cure an eye disease such as age-related macular degeneration.

### [Prior Art Document]

### [Patent Document]

Patent Document 1: Japanese Patent Application Laid-Open No. 2022-81491

### [Summary of the Invention]

### [Problem to be Solved by the Invention]

While intravitreal injection is an effective way to administer a medicine into the eye ball, it may cause a patient to suffer physical and mental strains, or may expose the patient to the possibility of infection. Therefore, development of a method by which a medicine can be administered in a less invasive manner is desired.

Further, a peptide that exhibits a physiological activity has been used recently for treatment of diseases of various kinds. Therefore, cases in which a peptide is used as a medicine for eye diseases may increase in the future.

In view of the foregoing, the present invention aims to provide a novel physiologically-active peptide derivative for treatment of eye diseases, a pharmaceutical composition, a nasal/nasal drop preparation, and a use of the physiologically-active peptide derivative.

### [Means for Solving the Problem]

The means for solving the problem includes the following embodiments.
<1> A physiologically-active peptide derivative for treatment of an eye disease, the physiologically-active peptide derivative having a physiologically-active peptide sequence and a functional sequence, and the functional sequence including a cell-penetration accelerating sequence and an endosomal-escape accelerating sequence.
<2> The physiologically-active peptide derivative according to <1>, further having a glycosylation molecule that includes a sugar chain.
<3> The physiologically-active peptide derivative according to <2>, wherein the glycosylation molecule is bound to a C-terminus side or an N-terminus side of the physiologically-active peptide sequence.
<4> The physiologically-active peptide derivative according to <2>, wherein a number of monosaccharide residues per sugar chain is from 5 to 20.
<5> The physiologically-active peptide derivative according to any one of <1> to <4>, wherein a number of amino acid residues of the physiologically-active peptide sequence is 200 or less.
<6> The physiologically-active peptide derivative according to any one of <1> to <5>, wherein the cell-penetration accelerating sequence is cationic.
<7> The physiologically-active peptide derivative according to any one of <1> to <6>, wherein half or more of a total number of amino acid residues of the cell-penetration accelerating sequence are basic amino acid residues.
<8> The physiologically-active peptide derivative according to any one of <1> to <7>, wherein the endosomal-escape accelerating sequence is an amino acid sequence selected from the group consisting of FFLIPKG, LILIG, FFG, FFFFG and FFFFFFG.
<9> The physiologically-active peptide derivative according to any one of <1> to <8>, which reaches an optic nerve or a retina via a lateral geniculate body.
<10> A pharmaceutical composition, comprising the physiologically-active derivative according to any one of <1> to <9> as an active ingredient.
<11> A nasal/nasal drop preparation, comprising the physiologically-active derivative according to any one of <1> to <9> as an active ingredient.
<12> Use of a physiologically-active peptide derivative for treatment of an eye disease, the physiologically-active peptide derivative having a physiologically-active peptide sequence and a functional sequence, and the functional sequence including a cell-penetration accelerating sequence and an endosomal-escape accelerating sequence.

### [Effect of the Invention]

According to the present invention, it is possible to provide a novel physiologically-active peptide derivative for treatment of eye diseases, a pharmaceutical composition, a nasal/nasal drop preparation, and a use of the physiologically-active peptide derivative.

### [Brief Explanation of the Drawings]

Fig. 1 is an image showing the result of fluorescent observation of mouse eye ball and optic nerve conducted in Example 1.
Fig. 2 is an image showing the result of fluorescent observation of mouse eye ball and optic nerve conducted in Example 2.
Fig. 3 is an image showing the result of fluorescent observation of mouse optic nerve conducted in Example 3.
Fig. 4 is an image showing the result of fluorescent observation of mouse optic nerve conducted in Example 4.
Fig. 5 is an image showing the result of fluorescent observation of mouse retina conducted in Example 5.
Fig. 6 is an image showing the result of fluorescent observation of mouse lateral geniculate body conducted in Example 6.
Fig. 7 is an image showing the result of fluorescent observation of mouse eye ball conducted in Example 7.
Fig. 8 is an image showing the result of fluorescent observation of mouse optic nerve conducted in Example 8.
Fig. 9 is an image showing the result of fluorescent observation of mouse retina conducted in Example 8.

### [Embodiments for Implementing the Invention]

In the following, embodiments of the invention are explained. The explanation and the embodiments described herein are intended to exemplify the invention, and do not limit the scope of the invention.

Numerical ranges indicated as "from A to B" described herein include A and B as a minimum value and a maximum value, respectively. In the amino acid sequence described therein, the left side thereof refers to the N-terminus and the right side refers to the C-terminus. The amino acid residue may be indicated by a one-letter abbreviation known in the art (for example, G for glycine residue).

The term "treatment" as described herein refers not only to an activity or an effect that quenches or alleviates a symptom, but also to an activity or an effect that suppresses aggravation of the symptom.

### <Physiologically-active peptide derivative>

The physiologically-active peptide derivative of the present invention has a physiologically-active peptide sequence, and a functional sequence that includes a cell-penetration accelerating sequence (hereinafter, also referred to as a cell penetrating peptide or CPP) and an endosomal-escape accelerating sequence (hereinafter, also referred to as a penetration accelerating sequence or PAS). The physiologically-active peptide derivative of the present invention is used for treatment of eye diseases.

When the present inventor nasally administered a mouse with a peptide, to which a functional sequence including a cell-penetration accelerating sequence and an endosomal-escape accelerating sequence was bound, migration of the peptide was observed toward the eye ball or optic nerve.

Namely, the physiologically-active peptide derivative of the present invention has an ability to cause a peptide as a medicine for eye diseases to act on the affected area by a non-invasive way of nasal administration. Therefore, the physiologically-active peptide derivative of the present invention is usable as a medicine for eye diseases that includes a peptide as an active ingredient.

The purpose of the physiologically-active peptide derivative is not particularly limited, as long as it employs a pharmaceutical effect that exhibits when a physiologically-active peptide sequence, included in the physiologically-active peptide derivative, acts on the eye ball or optic nerve. Examples of the purpose of the physiologically-active peptide derivative include treatment or improvement of various eye diseases, eye damages, eyestrain and a symptom caused by eyestrain.

Specific examples of the eye diseases to which the present invention can apply include optic neutritis, retrobulbar neutritis, neuromyelitis optica spectrum disorders, age-related macular degeneration, myopic choroidal neovascularization, branch retinal vein occlusion, central retinal vein occlusion, diabetic retinopathy, diabetic maculopathy, allergic conjunctivitis, viral conjunctivitis, bacterial conjunctivitis, infectious endophthalmitis, conjunctival chalaxation, chalazion, dacryocystitis, canaliculitis, orbital cellulitis, malignant tumor, retinitis pigmentosa, retinal break, vitreoretinal lymphoma, thyroid-associated ophthalmopathy, sarcoidosis, dry eye, Vogt-Koyanagi-Harada disease, macular hole, corneal infection, uveitis, glaucoma, optic neuropathy, cataract, and Behcet's disease. However, the scope of the present invention is not limited to these diseases.

The total number of amino acid residues included in the physiologically-active peptide derivative is not particularly limited. For example, the total number of amino acid residues included in the physiologically-active peptide derivative may be 250 or less, 200 or less, or 150 or less. The total number of amino acid residues included in the physiologically-active peptide derivative may be 10 or more, 20 or more, or 30 or more.

Each of the amino acid residues that constitutes the physiologically-active peptide derivative may be either L-form or D-form, as long as the effect of the present invention is achieved. The method for producing the physiologically-active peptide derivative is not particularly restricted, and may be a method of extracting from a living body or a natural substance, or may be prepared by a genetic engineering process or an organic synthetic process.

### (Physiologically-active peptide sequence)

The physiologically-active peptide sequence in the physiologically-active peptide derivative is not particularly limited, as long as it is derived from a peptide that acts on the eye ball or optic nerve and exhibits pharmaceutical effects. The method for combining the physiologically-active peptide sequence, the cell-penetration accelerating sequence and the endosomal-escape accelerating sequence is not particularly limited, and may be performed by a known method.

Specific examples of the peptide that acts on the eye ball or optic nerve and exhibits pharmaceutical effects include GLP-1 (number of amino acid residues: 23), GLP-2 (number of amino acid residues: 37), G-CSF (granulocyte colony stimulating factor, number of amino acid residues: 174 or 177), PEG-G-CSF (PEG-modified G-CSF, number of amino acid residues: 174 or 177), erythropoietin (number of amino acid residues: 165), PEG-modified erythropoietin (number of amino acid residues: 165), vasoactive intestinal peptide (VIP, number of amino acid residues: 28), oxytocin (number of amino acid residues: 9) and transforming growth factor TGF-β (number of amino acid residues: 112).

The number of amino acid residues included in the physiologically-active peptide sequence is not particularly limited. The total number of amino acid residues included in the physiologically-active peptide sequence may be from 5 to 200, from 5 to 170, from 9 to 120, from 9 to 70, or from 9 to 60. The total number of amino acid residues included in the physiologically-active peptide sequence may be from 5 or more, 10 or more, or 15 or more. The total number of amino acid residues included in the physiologically-active peptide sequence may be 200 or less, 170 or less, 120 or less, 70 or less, 60 or less, or 51 or less.

### (Cell-penetration accelerating sequence)

The cell-penetration accelerating sequence is an amino acid sequence derived from a peptide that exerts an activity to penetrate a cell membrane and migrates into a cell (cell-penetrating peptide).

Examples of the cell-penetrating peptide include oligoarginine (Rn, n is the number of arginine residues ranging from 6 to 12), oligolysine (Kn, n is the number of lysine residues ranging from 6 to 12), penetratin (RQIKIWFQNRRMKWKK, 16 amino acide residues, SEQ ID NO: 3), TAT (GRKKRRQRRR, 10 amino acid residues, SEQ ID NO: 4), mini penetratin (RRMKWKK, 7 amino acid residues, SEQ ID NO: 5), R9FC (RRRRRRRRRFFC, 12 amino acid residues, SEQ ID NO: 6), AIP6 (RLRWR, 5 amino acid residues, SEQ ID NO: 7), DPV3 (RKKRRRESRKKRRRES, 16 amino acid residues, SEQ ID NO: 8), DPV6 (GRPRESGKKRKRKRLKP, 17 amino acid residues, SEQ ID NO: 9), Pep-1 (KETWWETWWTEWSQPKKKRKV, 21 amino acid residues, SEQ ID NO: 10), MPG (GLAFLGFLGAAGSTMGAWSQPKKKRKV, 27 amino acid residues, SEQ ID NO: 11), Transportan (GWTLNSAGYLLGKINLKALAALAKKIL, 27 amino acid residues, SEQ ID NO: 12), MAP (KLALKALKALKAALKLA, 17 amino acid residues, SEQ ID NO: 13), W/R (RRWWRRWRR, 9 amino acid residues, SEQ ID NO: 14), CADY (GLWRALWRLLRSLWRLLWRA, 20 amino acid residues, SEQ ID NO: 15), EB-1 (LIRLWSHLIHIWFQNRRLKWKK, 22 amino acid residues, SEQ ID NO: 16), HRSV (RRIPNRRPRR, 10 amino acid residues, SEQ ID NO: 17), PTD-5 (RRQRRRTSKLMKR, 13 amino acid residues, SEQ ID NO: 18), TAT47-57 (YGRKKRRQRRR, 11 amino acid residues, SEQ ID NO: 19), TP2 (PLIYLRLLRGQF, 12 amino acid residues, SEQ ID NO: 20), TP10 (AGYLLGKINLHALAALAKKIL, 21 amino acid residues, SEQ ID NO: 21), a cationic sequence that binds to heparan, a cationic sequence that binds to RNA, and a cationic sequence that binds to DNA.

The cell-penetrating peptide that constitutes the cell-penetration accelerating sequence is preferably a cell-penetrating peptide that is positively charged, or a cell-penetrating peptide that is cationic. For example, a cationic cell-penetrating peptide formed of an amino acid sequence that is rich in basic amino acid residues such as arginine, lysine, histidine or tryptophan (for example, an amino acid sequence in which half or more of the total amino acid residues are basic amino acid residues) is preferred. Examples of such cell-penetrating peptides include oligoarginine, TAT derived from a Tat protein of human immunodeficiency virus 1 (HIV-1), penetratin, Pep-1, MPG, MAP, CADY, EB-1 and Transportan.

It is thought that a cell-penetrating peptide formed of an amino acid sequence that is rich in basic amino acid residues induces macropinocytosis as a form of endocytosis, which is a process of a cell to take in an extracellular substance. As such, it is thought that the physiologically-active peptide derivative is introduced into a cell in a more efficient manner.

While the number of amino acid residues in the cell-penetration accelerating sequence is not particularly limited, it is preferably from 5 to 27, for example. Further, the cell-penetration accelerating sequence is preferably a peptide in which half or more of the total amino acid residues are basic amino acid residues; more preferably a peptide that includes arginine residues as the basic amino acid residues; further preferably an oligoarginine formed of 6 to 12 arginine residues; yet further preferably an oligoarginine formed of 7 to 9 arginine residues; yet further preferably an oligoarginine formed of 8 arginine residues.

### (Endosomal-escape accelerating sequence)

It is thought that the endosomal-escape accelerating sequence shortens the time for the physiologically-active peptide derivative, which has been introduced into a cell, to remain in an endosome, and allows the physiologically-active peptide derivative to escape from an endosome in a shorter period. As a result, it is thought that the delivery of the physiologically-active peptide derivative to the eye ball or optic nerve, and the distribution of the physiologically-active peptide derivative in the eye ball or optic nerve, can be achieved in a shortened period.

The structure of the endosomal-escape accelerating sequence is not particularly limited, and examples thereof include amino acid sequences having an activity to promote endosomal escape such as FFLIPKG (SEQ ID NO: 22), LILIG (SEQ ID NO: 23), FFG, FFFFG (SEQ ID NO: 24) and FFFFFFG (SEQ ID NO: 25).

The position of the cell-penetration accelerating sequence and the endosomal-escape accelerating sequence in the physiologically-active peptide derivative is not particularly limited. For example, either one of the cell-penetration accelerating sequence or the endosomal-escape accelerating sequence may be disposed at a position closer to the physiologically-active peptide sequence. From the viewpoint of achieving the effect of the invention in a more efficient manner, it is preferred that the cell-penetration accelerating sequence is disposed at a position closer to the physiologically-active peptide sequence; and it is more preferred that the cell-penetration accelerating sequence is disposed at a position closer to the physiologically-active peptide sequence and the endosomal-escape accelerating sequence is disposed at an N-terminus side or a C-terminus side of the cell-penetration accelerating sequence.

### (Glycosylation molecule)

The physiologically-active peptide derivative preferably includes a glycosylation molecule, which includes a sugar chain, from the viewpoint of promoting the migration of the physiologically-active peptide derivative to the eye ball or optic nerve and distribution thereof in the eye ball or optic nerve.

The type of the sugar chain in the glycosylation molecule is not particularly limited. Specific examples of the sugar chain include N-linked sugar chains of high-mannose type, composite type or hybrid type (combination of high-mannose type and composite type), O-linked sugar chains, and proteoglycans such as mucin-type proteoglycan, heparan sulfate, chondroitin sulfate, keratan sulfate, hyaluronic acid and dermatan sulfate. Among these sugar chains, N-linked sugar chains are preferred, and composite-type N-linked sugar chains are more preferred.

The configuration of the sugar chain is not particularly limited, and may be a biantennary structure or other configurations (such as a branched configuration). Examples of the monosaccharide that constitutes a sugar chain include glucose, mannose, galactose, fructose, N-acetylglucosamine, N-acetylgalactosamine, N-acetylmannosamine, fucose, sialic acid, N-acetylneuramic acid, N-glycolylneuramic acid, deaminoneuramic acid, glucuronic acid, iduronic acid, galacturonic acid, xylose, ribose and deoxyribose. The monosaccharide that constitutes the sugar chain may be either D-form or L-form. The monosaccharide that constitutes the sugar chain may be either an α-anomer or a β-anomer.

The number of the glycosylation molecule included in the physiologically-active peptide derivative may be 1 or 2 or more. The number of the sugar chain included in a single glycosylation molecule may be 1 or 2 or more.

**In** the present disclosure, the number of the sugar chain is counted based on a basal portion of the sugar chain. Specifically, a group of monosaccharide residues stemming from a single basal portion is regarded as a single sugar chain.

The glycosylation molecule may be composed only of a sugar chain, or may be composed of a sugar chain and a portion other than the sugar chain. Namely, the sugar chain may bind to an amino acid residue that constitutes the physiologically-active peptide derivative in a direct manner or an indirect manner. **In** the present disclosure, each of a state in which a sugar chain directly binds to an amino acid residue that constitutes the physiologically-active peptide derivative, or a state in which a sugar chain indirectly binds to an amino acid residue that constitutes the physiologically-active peptide derivative, is regarded as a state in which the glycosylation molecule binds to an amino acid residue that constitutes the physiologically-active peptide derivative.

Examples of a state in which a sugar chain indirectly binds to an amino acid residue that constitutes the physiologically-active peptide derivative include a state in which a sugar chain binds to an amino acid residue that constitutes the physiologically-active peptide derivative via an amino acid residue such as a cysteine residue or an asparagine residue, or via a linker.

It is preferred that the glycosylation molecule binds to a portion such that the functions of the cell-penetration accelerating sequence or the endosomal-escape accelerating sequence are favorably maintained, and it is more preferred that the glycosylation molecule binds to the physiologically-active peptide sequence.

When the glycosylation molecule binds to the physiologically-active peptide sequence, the binding site for the glycosylation molecule is not particularly limited, as long as the stability or pharmaceutical activity of the physiologically-active peptide sequence is not lowered. Specifically, the binding site for the glycosylation molecule may be any of C-terminus side, N-terminus side, or other portions of the physiologically-active peptide sequence.

It is possible to substitute a portion of the physiologically-active peptide sequence with an amino acid residue to which a glycosylation molecule is likely to be bound, such as a cysteine residue or an asparagine residue, as long as the pharmaceutical activity of the physiologically-active peptide sequence is not affected. Alternatively, it is possible to introduce an amino acid residue at a terminus of the physiologically-active peptide sequence and allow the glycosylation molecule to bind thereto.

From the viewpoint of reducing the effect of a sugar chain on the function of functional sequences, the glycosylation molecule is preferably bound at a position at which an appropriate distance between the glycosylation molecule and the functional sequence can be secured.

Examples of the method for securing a distance between the glycosylation molecule and the functional sequence include a method of binding the glycosylation molecule to the C-terminus side of the physiologically-active peptide sequence, when the functional sequence is added to the N-terminus side of the physiologically-active peptide sequence; and a method of binding the glycosylation molecule to the N-terminus side of the physiologically-active peptide sequence, when the functional sequence is added to the C-terminus side of the physiologically-active peptide sequence.

Further examples of the method for securing a distance between the glycosylation molecule and the functional sequence include a method in which the glycosylation molecule further includes a linker through which the sugar chain is bound.

The type of the linker is not particularly limited, and examples thereof include an alkylene group and a polyethylene glycol (PEG) group.

When the linker is an alkylene group, the carbon number thereof is preferably from 3 to 15, more preferably from 4 to 12, further preferably from 5 to 10, from the viewpoint of securing an appropriate distance between the glycosylation molecule and the functional sequence.

The number of monosaccharide residues per sugar chain included in the glycosylation molecule is not particularly limited, and may be in a range of from 5 to 20, or from 5 to 15, for example.

From the viewpoint of improving the solubility of the physiologically-active peptide derivative with respect to an aqueous solvent, the number of monosaccharide residues per sugar chain is preferably 5 or more, more preferably 10 or more.

### (Spacer sequence)

The physiologically-active peptide derivative may further have a spacer sequence that is disposed between the physiologically-active peptide sequence and the functional sequence. When the spacer sequence is disposed between the physiologically-active peptide sequence and the functional sequence, the spacer sequence may exert an effect of preventing the activity of the physiologically-active peptide sequence from lowering or deteriorating.

When the physiologically-active peptide derivative has a spacer sequence, the spacer sequence may be disposed between the physiologically-active peptide sequence and the cell-penetration accelerating sequence, or may be disposed between the physiologically-active peptide sequence and the endosomal-escape accelerating sequence.

Generally, the cell-penetration accelerating sequence is formed of basic amino acid residues. Therefore, when the physiologically-active peptide sequence includes acidic amino acid residues, it may be possible to prevent the cell-penetration accelerating sequence and the physiologically-active peptide sequence from interacting with each other, thereby avoiding the lowering or degradation of the activity of the physiologically-active peptide sequence, by disposing a spacer sequence including neutral amino acid residues such as glycine by the number of from 1 to 10, preferably from 2 to 6.

The spacer sequence may include a lysine (K) residue. Lysine is an amino acid having a 4-aminobutyl group as a side chain. It is possible to dispose an alkylene group with a carbon number of 4 between the glycosylation molecule and the spacer sequence by bonding the glycosylation molecule to a terminal amino group in the 4-aminobutyl group. Therefore, it is possible to secure an appropriate distance between the glycosylation molecule and the functional sequence.

Examples of the spacer sequence including a lysine residue include those obtained by substituting one or more neutral amino acid residues such as glycine with a lysine residue, of a spacer sequence with the number of amino acid residues of from 1 to 10, preferably from 2 to 6, for example.

The physiologically-active peptide derivative may be subjected to various kinds of modification according to the intended purposes, in addition to the addition of a sugar chain. Examples of the modification include amino group modification (such as biotinylation, myristoylation, palmitoylation, acetylation and maleimidation), carboxy group modification (such as amidation and esterification), thiol group modification (such as farnesylation, geranylation, methylation and palmitoylation), hydroxy group modification (such as phosphorylation, sulfation, octanoylation, palmitoylation and palmitoleoylation, fluorescence labelling (such as FITC, FAM, rhodamine, BODIPY, NBD and MCA), pegylation, and introduciton of amino acids such as an unnatural amino acid or a D-amino acid. The modification may be performed on any of the physiologically-active peptide sequence, cell-penetration accelerating sequence, endosomal-escape accelerating sequence or spacer sequence.

The combination of the physiologically-active peptide sequence, cell-penetration accelerating sequence and endosomal-escape accelerating sequence as the constituents of the physiologically-active peptide derivative is not particularly limited, and may be selected depending on the intended purposes.

In an embodiment, the physiologically-active peptide derivative may have an endosomal-escape accelerating sequence, a cell-penetration accelerating sequence, a spacer sequence as necessary, and a physiologically-active peptide sequence, in this order from the N-terminus side.

In an embodiment, the physiologically-active peptide derivative may have an endosomal-escape accelerating sequence, a cell-penetration accelerating sequence, a spacer sequence as necessary, and a physiologically-active peptide sequence, in this order from the C-terminus side.

In the foregoing configurations, the endosomal-escape accelerating sequence may be selected from FFLIPKG, LILIG, FFG, FFFFG or FFFFFFG.

In the foregoing configurations, the cell-penetration accelerating sequence may be selected from oligoarginines (Rn, n=6-12).

In the foregoing configurations, the spacer sequence may be selected from a sequence composed of a glycine residue (for example, Gn, n=2-6), a sequence composed of a glycine residue and a cysteine residue (for example, GCG) and a sequence composed of a glycine residue and a lysine residue (for example, GKG).

### <Pharmaceutical composition>

The pharmaceutical composition of the present invention includes a physiologically-active peptide derivative as an active ingredient. By including a physiologically-active peptide derivative as an active ingredient, the pharmaceutical composition can be delivered to the eye ball or optic nerve in a favorable manner, and exhibit a pharmacological effect in an efficient manner. Therefore, for example, the pharmaceutical composition is suitable for the therapy of eye diseases in which daily medication at home is required. Accordingly, preferred examples of formulation of the pharmaceutical composition include nasal/nasal drop preparations.

The details and the preferred embodiments of the physiologically-active peptide derivative included in the pharmaceutical composition are as described above. From the viewpoint of the ability to be delivered to the eye ball or optic nerve, the method for using the pharmaceutical composition is preferably intranasal/nasal drop administration.

The pharmaceutical composition may include a component other than the physiologically-active peptide derivative. Examples of the component other than the physiologically-active peptide derivative include a medium and a formulation additive that are used for the preparation of a pharmaceutical composition. Specific examples of the formulation additive include a diluent, a disintegrant, a binder, a lubricant, a surfactant, a buffer, a solubilizing agent, a stabilizer, a tonicity agent, a suspending agent, an emulcifier, a solvent, a thickner, a mucolytic agent, a humectant and a preservative. The dosage amount of the pharmaceutical composition may be selected according to the type of disease, the symptomatic state, weight or age of the patient, the administration form and the like.

The pharmaceutical composition of the present invention is particularly suitable as an nasal/nasal drop preparation. Therefore, an embodiment of the present invention is a use of the present invention for nasal/nasal drop administration.

### <Nasal/nasal drop preparation>

The nasal/nasal drop preparation of the present inveniton includes a physiologically-active peptide derivative as described above as an active ingredient. By including a physiologically-active peptide derivative as an active ingredient, the nasal/nasal drop preparation can be delivered to the eye ball or optic nerve in a favorable manner, and exhibit a pharmacological effect in an efficient manner. Further, since the nasal/nasal drop preparation can be administered is a less invasive manner, it is useful for the therapy of a disease that requires daily medication at home.

The nasal/nasal drop preparation may include a component other than the physiologically-active peptide derivative. Examples of the component other than the physiologically-active peptide derivative include a medium and a formulation additive that may be used for the preparation of the pharmaceutical composition, such as those as described above.

The embodiments of the present invention include a use of the pharmaceutical composition including a physiologically-active peptide derivative as an active ingredient, as described above, for intranasal administration. The details and the preferred embodiments of the physiologically-active peptide derivative and the pharmaceutical composition for the use are as described above.

### <Therapeutic method for eye diseases >

The embodiments of the present invention include a therapeutic method for eye diseases, the method including administrating, to a patient, the physiologically-active peptide derivative or the pharmaceutical composition as mentioned above. The details and preferred embodiments of the physiologically-active peptide derivative or the pharmaceutical composition in the therapeutic method are as described above.

Specific examples of the eye diseases to be treated by the therapeutic method include the eye diseases as mentioned above to which the physiologically-active peptide derivative is applied.

While the method for administrating the physiologically-active peptide derivative or the pharmaceutical composition to a patient is not particularly limited, it is preferably intranasal administration.

### [Examples]

The present invention is explained in further detail by referring to the following examples. The materials, amounts, rates, procedures and the like may be modified without departing from the scope of the invention. Therefore, the scope of the present invention should not be construed in a restricted manner by the examples as described below.

In the following examples, GLP-2 used for the preparation of the physiologically-active peptide derivative is a peptide having an amino acid sequence of
HADGSFSDEMNTILDNLAARDFINWLIQTKITD (SEQ ID NO: 1) and GLP-1 used for the preparation of the physiologically-active peptide derivative is a peptide having an amino acid sequence of HAEGTFTSDVSSYLEGQAAKEFIAWLVKGR-NH₂ (activity type: 7-36 amide, SEQ ID NO: 2).

### <Example 1: migration of GLP-2 derivative to eye ball and optic nerve>

A GLP-2 derivative (C-terminus 11-sugar) in which an endosomal-escape accelerating sequence (PAS: FFLIPKG), a cell-penetration accelerating sequence (CPP: RRRRRRRR), a spacer sequence (GG), and an amino acid sequence derived from GLP-2 as a physiologically-active peptide sequence were disposed in this order from the N-terminus side was prepared by a conventional method. As the physiologically-active peptide sequence, an amino acid sequence of GLP-2 to which a sugar chain formed of 11 monosaccharide residues was added at the C-terminus via a cysteine residue was used. The configuration of the GLP-2 derivative is shown below.

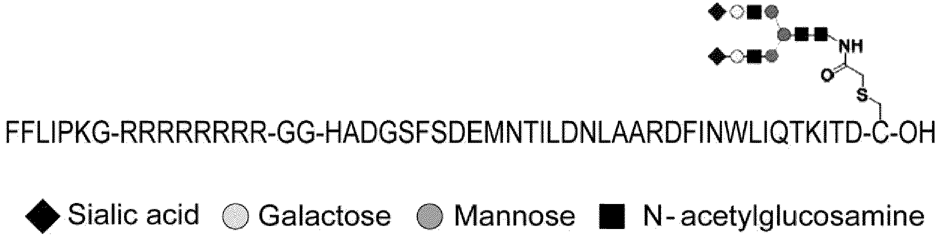

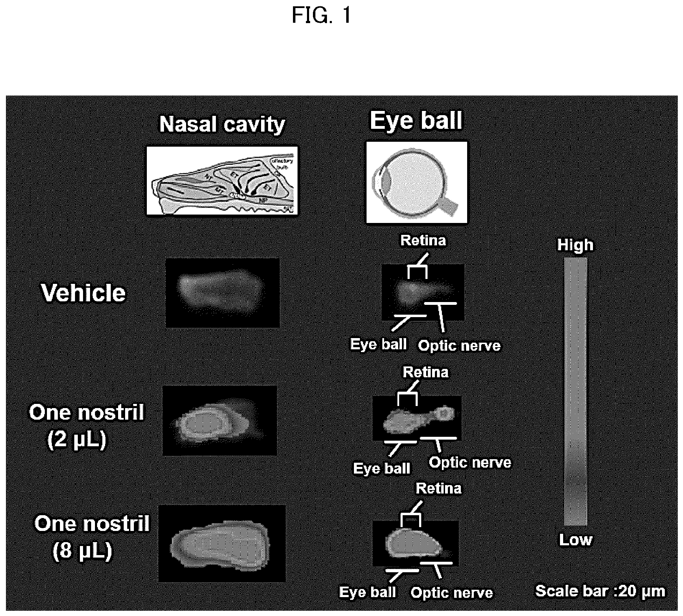

Intranasal administration was performed to mice (male ddY, 7 week-old, hereinafter the same) with 16% DMSO (Vehicle) or a DMSO solution of ICG-labeled GLP-2 derivative (3.0 nmol/mouse or 12.0 nmol/mouse). The eye ball was isolated 20 minutes after the administration, and subjected to infiltration fixation overnight with a 4% PFA solution. Thereafter, the eye ball was rinsed with 1×PBS and placed on a dish, and the measurement was conducted using an optical imaging device (Clairvivo OPT plus; Shimadzu, Kyoto, Japan, hereinafter the same). The measurement was conducted at an excitation wavelength of 785 nm, a fluorescence wavelength of 849 nm, and an exposure time of 6 sec. The results are shown in FIG. 1.

As shown in FIG. 1, it was confirmed that the GLP-2 derivative administered in an intranasal manner migrated to the eye ball and the optic nerve.

### <Example 2: migration of GLP-1 derivative to eye ball and optic nerve>

A GLP-1 derivative (C-terminus 11-sugar) in which a sugar chain was added to the C-terminus side of GLP-1 was prepared in the same manner as Example 1, except that the physiologically-active peptide sequence was changed from GLP-2 to GLP-1. The configuration of the GLP-1 derivative is shown below.

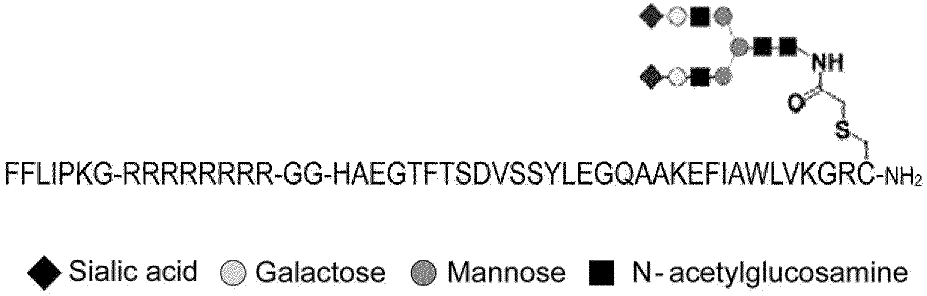

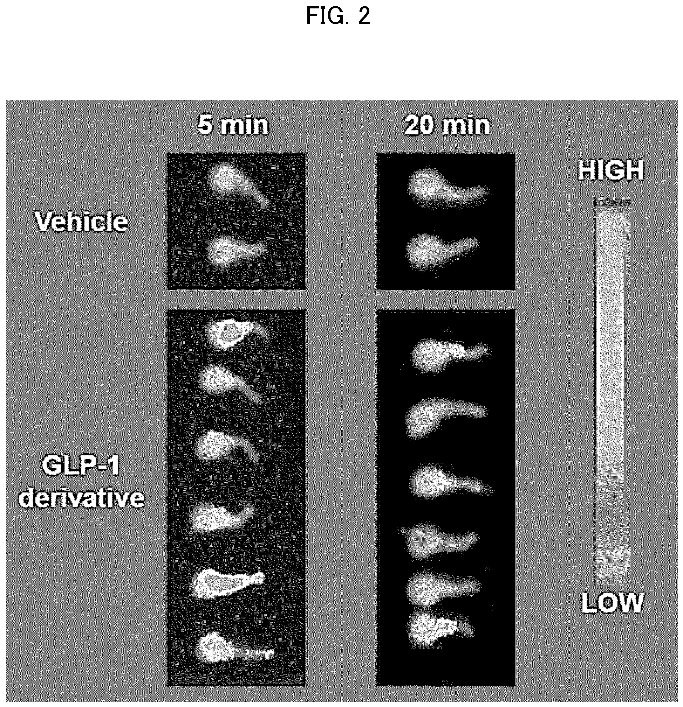

Intranasal administration was performed to mice with 16% DMSO (Vehicle) or a DMSO solution of ICG-labeled GLP-1 derivative (0.9 nmol/mouse). The eye ball was isolated 5 minutes or 20 minutes after the administration, and subjected to infiltration fixation overnight with a 4% PFA solution. Thereafter, the eye ball was rinsed with 1×PBS and placed on a dish, and the measurement was conducted using an optical imaging device. The measurement was conducted at an excitation wavelength of 785 nm, a fluorescence wavelength of 849 nm, and an exposure time of 6 sec. The results are shown in FIG. 2.

As shown in FIG. 2, it was confirmed that the GLP-1 derivative (C-terminus 11-sugar) administered in an intranasal manner migrated to the eye ball and the optic nerve.

### <Example 3: distribution of GLP-2 derivative in optic nerve>

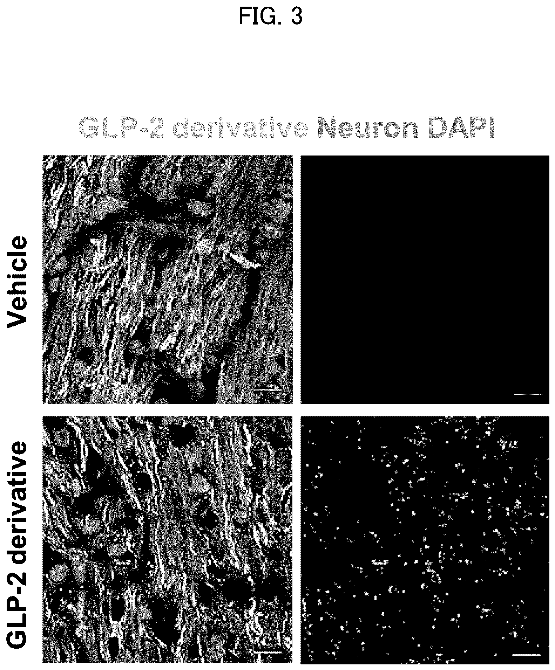

Intranasal administration was performed to mice with PBS (Vehicle) or a PBS solution of ICG-labeled GLP-2 derivative used in Example 1 (3.0 nmol/mouse). The eye ball was isolated 20 minutes after the administration, and subjected to infiltration fixation overnight with a 4% PFA solution. On the next day of the isolation, 4% PFA was substituted with 30% sucrose overnight (4°C). Thereafter, a frozen section of the optic nerve with a thickness of 10 µm was prepared using a cryostat (CM3050S; Leica Microsystems, WetZlar, Germany, hereinafter the same). The section was placed on a dish, added with a blocking buffer, and subjected to blocking at room temperature for 30 minutes. Thereafter, the section was added with a primary antibody solution including Neuro-Chrom^{™} Pan Neuronal Marker Antibody-Rabbit (1:500) and GLP-2 polyclonal Antibody (1:200) diluted with a blocking buffer, and incubated at room temperature for 2 hours. The section was rinsed with 1×PBS for 3 times, added with a secondary antibody solution including Alexa Fluor (registered tradename) 568 Goat Anti-Mouse IgG H&L and 40 ng/ml DAPI, diluted by 500 times with 1% BSA/PBS solution, and incubated at room temperature for 1 hour. The section was rinsed with 1×PBS for 3 times and mounted, and the fluorescent observation was conducted using a confocal laser microscope (TCS SP8; Leica, WetZlar, Germany, hereinafter the same) with a software (Leica Application Suite X Software; Leica, WetZlar, Germany, hereinafter the same). The results are shown in FIG. 3.

As shown in FIG. 3, it was confirmed that the GLP-2 derivative (C-terminus 11-sugar, corresponding to dot-shaped fluorescence) administered in an intranasal manner distributed in the optic nerve.

### <Example 4: distribution of GLP-1 derivative in optic nerve>

A PAS-CPP-GLP-1 derivative (no sugar) in which an endosomal-escape accelerating sequence (PAS: FFLIPKG), a cell-penetration accelerating sequence (CPP: RRRRRRRR), a spacer sequence including a cysteine residue (GCG), and an amino acid sequence derived from GLP-1 as a neuropeptide sequence were disposed in this order from the N-terminus side was obtained. The configuration of the GLP-1 derivative is shown below.

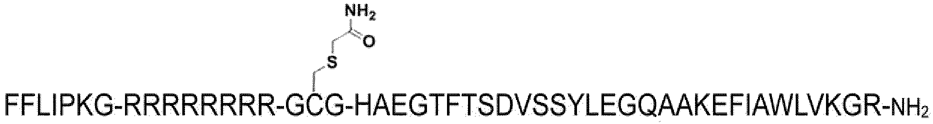

Subsequently, a PAS-CPP-GLP-1 derivative (N-terminus 11-sugar) was obtained by allowing a sugar chain (11-sugar) to bind to the cysteine residue of the spacer sequence. The configuration of the GLP-1 derivative is shown below.

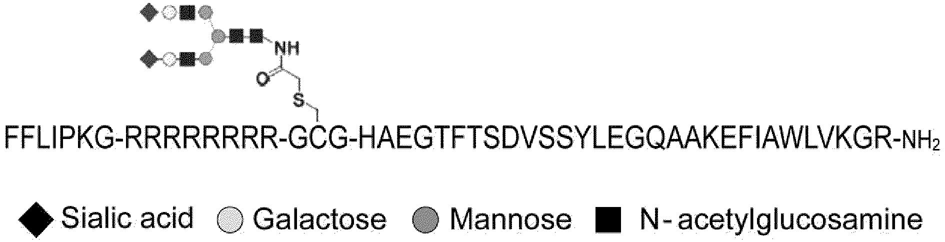

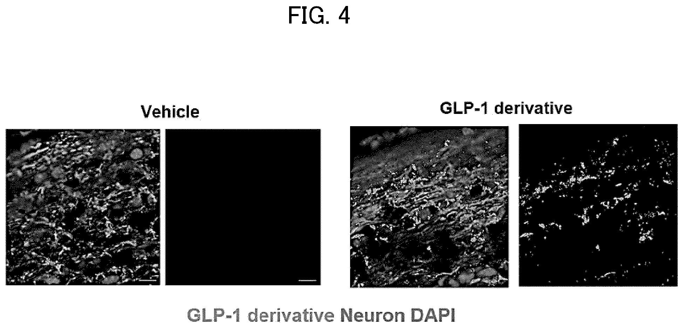

Intranasal administration was performed to mice with PBS (Vehicle) or a PBS solution of GLP-1 derivative (N-terminus 11-sugar ) (3.6 nmol/mouse). The eye ball was isolated 20 minutes after the administration, and a frozen section of the optic nerve was prepared and fluorescent observation was performed in the same manner as Example 3. The results are shown in FIG. 4.

As shown in FIG. 4, it was confirmed that the GLP-1 derivative (N-terminus 11-sugar, corresponding to dot-shaped fluorescence) administered in an intranasal manner distributed in the optic nerve.

### <Example 5: distribution of GLP-1 derivative in retina >

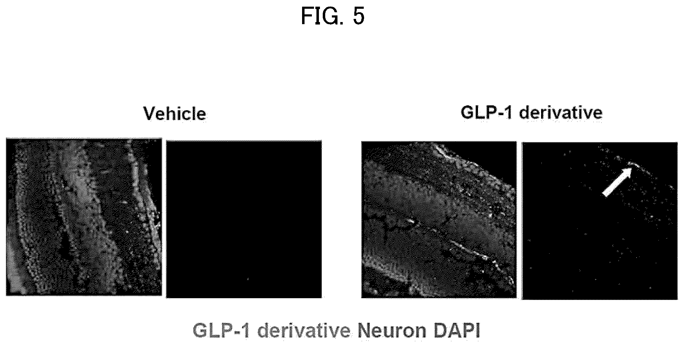

Intranasal administration was performed to mice with PBS (Vehicle) or a PBS solution of GLP-1 derivative (N-terminus 11-sugar) (3.6 nmol/mouse). The eye ball was isolated 20 minutes after the administration, and a frozen section of the retina was prepared and fluorescent observation was performed in the same manner as Example 4. The results are shown in FIG. 5.

As shown in FIG. 5, it was confirmed that the GLP-1 derivative (N-terminus 11-sugar, corresponding to fluorescence indicated by arrow) administered in an intranasal manner distributed in the retina.

### <Example 6: distribution of GLP-2 derivative in lateral geniculate body>

A GLP-2 derivative (N-terminus 11-sugar) was prepared in the same manner as Example 4, except that the physiologically-active peptide sequence was changed from GLP-2 to GLP-1. The configuration of the GLP-2 derivative is shown below.

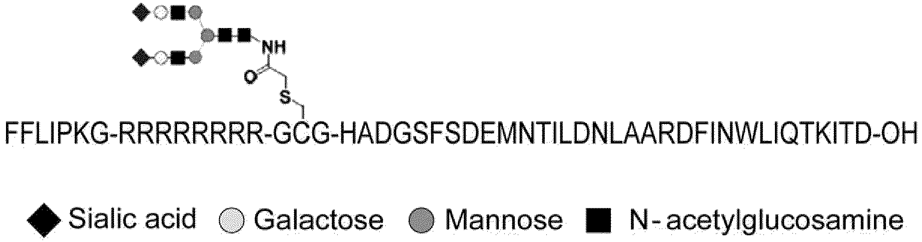

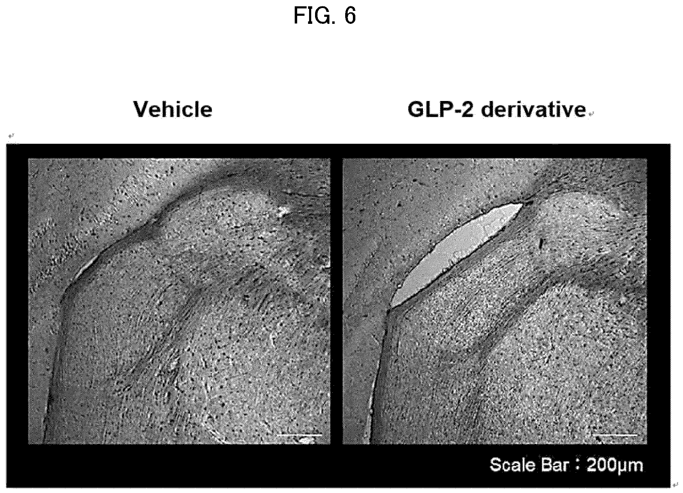

Intranasal administration was performed to mice with PBS or a PBS solution of GLP-2 derivative (3.0 nmol/mouse). The brain was isolated 5 minutes after the administration, and subjected to infiltration fixation overnight with a 4% PFA solution. On the next day of the isolation, 4% PFA was substituted with 30% sucrose overnight (4°C). Thereafter, a frozen section of the lateral geniculate body with a thickness of 30 µm was prepared using a cryostat. The section was placed on a slide glass, and a liquid blocker was applied in a circular manner around the section. A blocking buffer was added into the circle and subjected to blocking at room temperature for 30 minutes. Thereafter, the section was added with a primary antibody solution including GLP-2 polyclonal Antibody diluted with a blocking buffer by 200 times, and incubated at room temperature for 1 hour. The section was rinsed with 1×PBS for 3 times, added with a secondary antibody solution including Alexa Fluor (registered tradename) 568 Goat Anti-Mouse IgG H&L diluted by 500 times with 1% BSA/PBS solution, and incubated at room temperature for 1 hour. The section was rinsed with 1×PBS for 3 times and mounted with ProLong^{™} Diamond Antifade Mountant. After confirming the solidification of the mountant, fluorescent observation was conducted using a confocal laser microscope with a software. The results are shown in FIG. 6.

As shown in FIG. 6, fluorescence corresponding to the GLP-2 derivative administered in an intranasal manner was observed, indicating that the GLP-2 derivative administered in an intranasal manner migrated to the eye ball and the optic nerve via the lateral geniculate body. The foregoing findings discover a new route with no previous reports, i.e., the peptide administered in an intranasal manner migrates to the eye ball and the optic nerve via the lateral geniculate body in the thalamus, thereby suggesting a possible application of the peptide administered in an intranasal manner to the treatment of eye diseases such as optic neuritis or optic neuropathy.

### <Example 7: migration to eye ball of GLP-2 derivative with sugar chain bound via linker>

A GLP-2 derivative (C6-linker N-terminus 11-sugar) was prepared in the same manner as Example 6, except that the spacer sequence (GCG) was changed to a spacer sequence (GKG), and that a molecule including a sugar chain (11-sugar) and an alkylene group with a carbon number of 6 as a linker was bound to the lysine (K) residue in the spacer sequence. The configuration of the GLP-2 derivative is shown below.

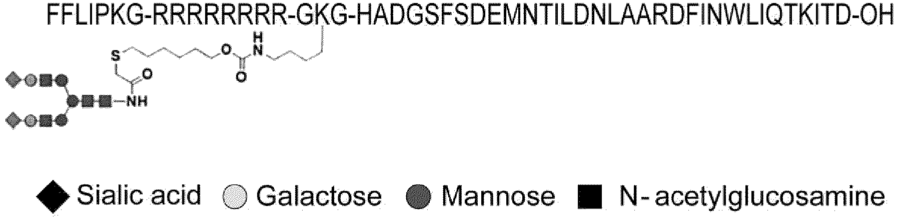

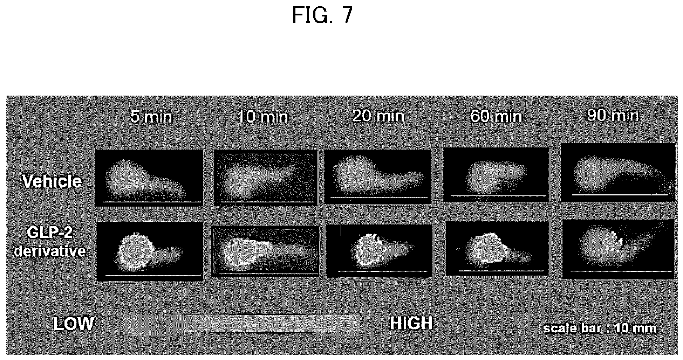

Intranasal administration was performed to mice with 16% DMSO (Vehicle) or a DMSO solution of ICG-labeled GLP-2 derivative (C6-linker N-terminus 11-sugar) (3.0 nmol/mouse). The eye ball was isolated 5 minutes, 10 minutes, 20 minutes, 60 minutes or 90 minutes after the administration, and subjected to infiltration fixation overnight with a 4% PFA solution. Thereafter, the eye ball was rinsed with 1×PBS and placed on a dish, and the measurement was conducted using an optical imaging device. The measurement was conducted at an excitation wavelength of 785 nm, a fluorescence wavelength of 849 nm, and an exposure time of 6 sec. The results are shown in FIG. 7.

As shown in FIG. 7, it was confirmed that the GLP-2 derivative (C6-linker N-terminus 11-sugar) administered in an intranasal manner migrated to the eye ball within 5 minutes from the administration. Further, it was confirmed that the GLP-2 derivative (C6-linker N-terminus 11-sugar) remained at the eye ball even after 90 minutes from the administration.

### <Example 8: migration to optic nerve and retina of GLP-2 derivative with sugar chain bound via linker>

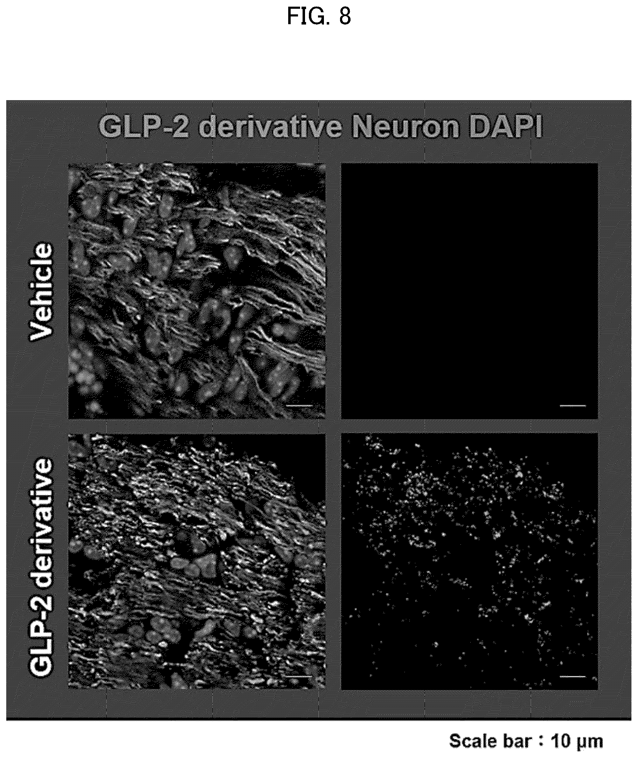

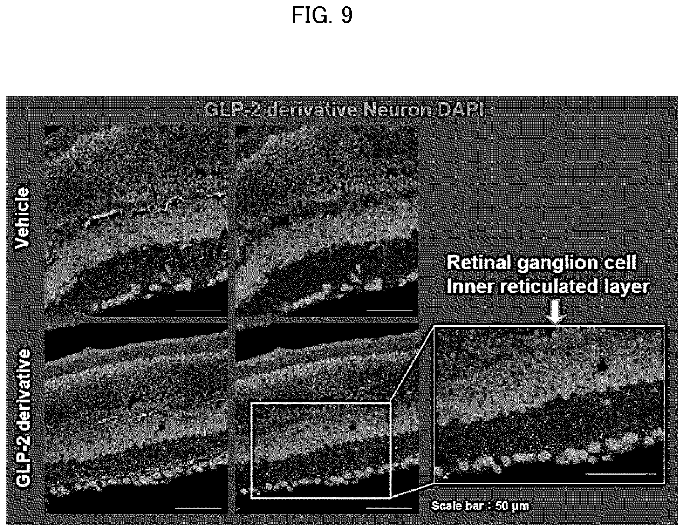

Intranasal administration was performed to mice with PBS (Vehicle) or a PBS solution of GLP-2 derivative (C6-linker N-terminus 11-sugar) (3.0 nmol/mouse). The eye ball was isolated 20 minutes after the administration, and subjected to infiltration fixation overnight with a 4% PFA solution. On the next day of the isolation, 4% PFA was substituted with 30% sucrose overnight (4°C). Thereafter, frozen sections of the optic nerve and the retina with a thickness of 10 µm were prepared using a cryostat. The section was placed on a dish, added with a blocking buffer, and subjected to blocking at room temperature for 30 minutes. Thereafter, the section was added with a primary antibody solution including Neuro-Chrom^{™} Pan Neuronal Marker Antibody-Rabbit diluted with a blocking buffer (1:500) and GLP-2 polyclonal Antibody diluted with a blocking buffer (1:200), and incubated at room temperature for 2 hours. The section was rinsed with 1×PBS for 3 times, added with a secondary antibody solution including Alexa Fluor (registered tradename) 568 Goat Anti-Mouse IgG H&L diluted by 500 times with 1% BSA/PBS solution, and 20 µg/ml of DAPI, and incubated at room temperature for 1 hour. The section was rinsed with 1×PBS for 3 times and mounted, and the fluorescent observation was conducted using a confocal laser microscope with a software. The results of the fluorescent observation at a portion corresponding to the optic nerve are shown in FIG. 8, and the results of the fluorescent observation at a portion corresponding to the retina are shown in FIG. 9.

As shown in FIG. 8 and FIG. 9, it was confirmed that the GLP-2 derivative (C6-linker N-terminus 11-sugar, corresponding to dot-shaped fluorescence) administered in an intranasal manner migrated to the retinal ganglion cell or the inner reticulated layer within 20 minutes from the administration.

The disclosure of Japanese Patent Application No. 2022-180381 is incorporated herein by reference in its entirety. All publications, patent applications, and technical standards mentioned in the present specification are incorporated herein by reference to the same extent as if each individual publication, patent application, or technical standard was specifically and individually indicated to be incorporated by reference.

## Claims

1. A physiologically-active peptide derivative for treatment of an eye disease, the physiologically-active peptide derivative having a physiologically-active peptide sequence and a functional sequence, and the functional sequence including a cell-penetration accelerating sequence and an endosomal-escape accelerating sequence.

2. The physiologically-active peptide derivative according to claim 1, further having a glycosylation molecule that includes a sugar chain.

3. The physiologically-active peptide derivative according to claim 2, wherein the glycosylation molecule is bound to a C-terminus side or an N-terminus side of the physiologically-active peptide sequence.

4. The physiologically-active peptide derivative according to claim 2, wherein a number of monosaccharide residues per sugar chain is from 5 to 20.

5. The physiologically-active peptide derivative according to claim 1, wherein a number of amino acid residues of the physiologically-active peptide sequence is 200 or less.

6. The physiologically-active peptide derivative according to claim 1, wherein the cell-penetration accelerating sequence is cationic.

7. The physiologically-active peptide derivative according to claim 1, wherein half or more of a total number of amino acid residues of the cell-penetration accelerating sequence are basic amino acid residues.

8. The physiologically-active peptide derivative according to claim 1, wherein the endosomal-escape accelerating sequence is an amino acid sequence selected from the group consisting of FFLIPKG, LILIG, FFG, FFFFG and FFFFFFG.

9. The physiologically-active peptide derivative according to claim 1, which reaches an optic nerve or a retina via a lateral geniculate body.

10. A pharmaceutical composition, comprising the physiologically-active derivative according to any one of claim 1 to claim 9 as an active ingredient.

11. An intranasal/nasal drop preparation, comprising the physiologically-active derivative according to any one of claim 1 to claim 9 as an active ingredient.

12. Use of a physiologically-active peptide derivative for treatment of an eye disease, the physiologically-active peptide derivative having a physiologically-active peptide sequence and a functional sequence, and the functional sequence including a cell-penetration accelerating sequence and an endosomal-escape accelerating sequence.
